# EUROPEAN PATENT APPLICATION

(11) **EP 4 782 056 A1**
(43) Date of publication of application: **29.07.2026**
(21) Application number: 25153793.2
(22) Date of filing: 24.01.2025
(51) Int. Cl.: A61N 5/10, A61G 13/04

(54) **PATIENT COUCH FOR POSITIONING AND/OR SUPPORTING A PATIENT DURING RADIATION THERAPY**

(71) Applicant: Varian Medical Systems, Inc., Palo Alto, CA 94304 (US)
(72) Inventor: Barthel, Markus, 95478 Kemnath (DE); Baumann, Berthold, 95506 Kastl (DE); Buchbinder, Stefan, 95469 Speichersdorf (DE); Buchenauer, Andreas, 95445 Bayreuth (DE); Eigner, Daniel, 92681 Erbendorf (DE); Hruschka, Klaus, 92681 Erbendorf (DE); Kilian, Katrin, 92637 Weiden (DE); Knodt, Konstantin, 92681 Erbendorf (DE); Neuber, Wolfgang, 92676 Eschenbach i. d. Opf. (DE); Plannerer, Patrick, 95478 Kulmain (DE); Schmälzle, Thomas, 95448 Bayreuth (DE); Stock, Johannes, 92717 Reuth bei Erbendorf (DE)
(74) Representative: Siemens Healthineers Patent Attorneys

(57) **Abstract**

The invention relates to a patient couch (2) for positioning and/or supporting a patient for radiotherapy, comprising:
- a couch board section (7) for accommodating the patient,
- a base section (8) for attachment to the floor,
- a roll-pitch assembly (11) arranged between the base section (8) and the couch board section (7), designed to pivot the couch board section (7) about a roll axis (17) and/or a pitch axis (15),
characterized by a cover unit (19) for concealing the roll-pitch assembly (11) and/or as a pinch protection for hands, wherein the cover unit (19) forms a shell cover and comprises at least two cover shells (20), wherein one of the cover shells (20) forms a roll cover shell (d) and another of the cover shells (20) forms a pitch cover shell (20b), wherein the roll cover shell (20b) is designed and/or arranged to compensate a roll movement of the roll-pitch assembly (11), wherein the pitch cover shell (20b) is designed and/or arranged to compensate a pitch movement of the roll-pitch assembly (11).

## Description

*Independent of the grammatical term usage, individuals with male, female or other gender identities are included within the term.*

The present invention relates to a patient couch for positioning and/or supporting a patient during radiation therapy. The patient couch comprises a couch board section for accommodating the patient, a base section for attachment to the floor, a roll-pitch assembly arranged between the base section and the couch board section, designed to pivot the couch board section about a roll axis and/or a pitch axis.

Patient couches are widely used in various medical fields for the transport and positioning of patients during imaging, treatment, and/or inpatient care. Additionally, patient couches play a critical role in radiation therapy, where precise positioning and stability are essential to ensure accurate treatment delivery.

Patient couches employed in radiation therapy must be particularly robust against radiation and enable highly precise positioning of the patient. This is critical to ensure that the radiation is accurately directed at the target area while sparing the surrounding healthy tissue as much as possible. In radiation therapy, it is customary for patient couches to be designed to offer high stability and precision. The couches must be capable of holding the patient in a fixed position to minimize movement during treatment. This is often achieved through the use of fixation devices and specialized positioning aids. For patient positioning, the couches include mechanisms for horizontal and vertical adjustments as well as mechanisms for tilting (pitch, roll).

The mechanisms for tilting, swiveling, and positioning the patient must be designed to prevent accidental interference. This is important to avoid pinching injuries to hands, patients, or staff. Safety covers and protective devices are therefore integral parts of the design to minimize the risk of injury. To prevent such interferences covers may be used.

Covers are used also for aesthetic reasons but, more importantly, for regulatory compliance. These covers must be particularly easy to clean, hygienic, and suitable for use as medical devices. At the same time, they must be resistant to harsh ionizing radiation to ensure their functionality and integrity throughout the product's lifetime.

Covers are used also for aesthetic reasons but, more importantly, for regulatory compliance. These covers must be particularly easy to clean, hygienic, and suitable for use as medical devices. At the same time, they must be resistant to harsh ionizing radiation to ensure their functionality and integrity throughout the product's lifetime. Moreover, the covers should be designed for easy assembly and disassembly to facilitate maintenance and service, and their movement should be smooth and quiet, without any abnormal noises.

Patient couches in the prior art often impose significant constraints on the available treatment volume, which can hinder the effectiveness and flexibility of radiation therapy procedures. This limitation is primarily caused by physical collisions between the couch covers and the gantry, or between the couch covers and the gantry covers, particularly when the couch is adjusted for specific roll and pitch positions. Such collisions not only restrict the range of possible patient positioning but may also disrupt the smooth operation of the equipment. Additionally, existing covers for roll-pitch-capable couches are frequently bulky, adding unnecessary complexity to the system. They are also often difficult to clean, which poses challenges for maintaining hygiene standards, especially in a clinical environment where strict sanitation is required.

The objective of the present invention is to provide a patient couch specifically designed for use in radiation therapy that addresses these issues. The goal is to develop a couch that significantly improves upon the limitations of the prior art, offering enhanced compatibility with treatment equipment and expanded positioning flexibility. Moreover, the invention seeks to create a solution that incorporates a more compact and streamlined design for the covers, ensuring ease of cleaning and compliance with stringent hygiene requirements, while maintaining robustness against the effects of ionizing radiation.

The objective is achieved by the present invention, particularly through the patient couch, the use of the patient couch, and the radiotherapy device. Preferred and/or advantageous embodiments are derived from the dependent claims, the description, and the accompanying figures.

The patient couch is configured for positioning and/or supporting a patient or an object for a radiotherapy. The patient couch is preferably stationary. Alternatively, the patient couch can be mobile. The patient can be positioned on the patient couch, particularly in a lying position. Specifically, the patient couch includes means for securing and/or fixing the patient. Examples of such means include straps, clamps, and customized cushions that conform to the patient's body shape to ensure stability and comfort during treatment.

The patient couch can be part of a radiotherapy system and/or can be used or positioned near the treatment area of a LINAC (Linear Accelerator). The patient couch is particularly designed for height adjustment, allowing the patient to be lowered or raised as needed. Furthermore, the patient couch is preferably designed to move the patient and/or the couch board section horizontally, for example, in two orthogonal directions, such as longitudinal and transverse directions.

This horizontal movement capability is crucial for precise positioning, ensuring that the patient is accurately aligned with the radiation beam. The combination of vertical and horizontal adjustments allows for fine-tuning the patient's position, which is essential for targeting the treatment area while minimizing exposure to surrounding healthy tissues.

The patient couch comprises a couch board section designed to accommodate the patient. This section, often referred to as the patient support board or includes a patient support board. The couch board section, especially the patient support board, is configured for providing a stable and comfortable surface for the patient during radiotherapy treatments. couch support boards can be made from various materials, each chosen for its specific properties. Preferably the couch board section, especially the patient support board, is based on carbon fiber composites, which provide strength, rigidity, and lightweight. Carbon fiber is particularly advantageous in radiotherapy settings because it is radiolucent, meaning it does not interfere with the radiation beams, allowing for precise targeting of the treatment area. Furthermore, couch board section, especially the patient support board, can be based or made of high-density polyethylene and specialized medical-grade plastics, which offer durability and ease of cleaning.

The couch board section, especially the patient support board, is constructed to ensure minimal deflection and maximum stability. They couch board section, especially the patient support board, preferably features a modular design, allowing for easy attachment of accessories such as immobilization devices and positioning aids. This modularity is essential for customizing the setup to meet the specific needs of each patient and treatment plan.

Preferably, the couch board section, especially the patient support board, comprises an indexing systems that allow for reproducible positioning, ensuring that the patient can be placed in the exact same position for each treatment session. The couch board section, especially the patient support board, can comprise markers, especially radiopaque markers, for positioning and/or position verification. Optionally, the couch board section may have built-in channels or slots for securing straps and other fixation devices, enhancing patient stability and safety during treatment.

The patient couch includes a base section designed for attachment to the floor. This base section is configured for providing stability and support for the entire couch structure. It can be constructed to form a secure connection with the floor, ensuring that the couch remains stationary during patient positioning and treatment.

The base section can serve as a foundation or pedestal for the patient couch. It may include a lifting mechanism, such as a scissor lift, to enable vertical adjustment of the patient couch. This allows the patient to be raised or lowered to the desired height, facilitating precise alignment with the radiation beam.

To enhance safety and functionality, the base section is preferably enclosed. This enclosure protects the internal mechanisms from dust and debris, and also prevents accidental contact with moving parts, reducing the risk of injury to patients and medical staff. The enclosure can be made from durable materials that are easy to clean and disinfect, ensuring compliance with medical hygiene standards. Preferably, the base section may incorporate features for horizontal movement, allowing the patient couch to be shifted in two orthogonal directions, such as longitudinal and transverse directions.

The design of the base section also takes into account the need for robustness and durability. It must be capable of supporting the weight of the patient and any additional equipment used during treatment. The materials used in its construction should be resistant to wear and tear, as well as to the effects of ionizing radiation, ensuring long-term reliability and safety.

The patient couch includes a roll-pitch assembly positioned between the base section and the couch board section. The a roll-pitch assembly is designed to pivot the couch board section about a roll axis and/or a pitch axis, providing precise adjustments to the patient's position during radiotherapy. The roll-pitch assembly allows for the rotation of the couch board section around two primary axes: the roll axis and the pitch axis. This capability allows achieving optimal patient alignment with the radiation beam, ensuring accurate targeting of the treatment area while minimizing exposure to surrounding healthy tissues.

The roll-pitch assembly can be constructed using various mechanical components, such as bearings, actuators, and control systems. These components work together to provide smooth and precise movements. The a roll-pitch assembly may include motorized actuators that enable automated adjustments, controlled via a user interface or integrated software.

Preferably, the roll-pitch assembly is configured as a cardanic mechanism and/or a gimbal mechanism. For example, the roll-pitch assembly involves the use of a gimbal mechanism. The roll-pitch assempbly may comprise two intersecting and orthogonally arranged rotational axes. This gimbal setup, also known as a Cardanic suspension, allows for independent rotation around both the roll and pitch axes. The gimbal mechanism ensures that the couch board section can be precisely tilted and rotated, providing enhanced flexibility in patient positioning.

The patient couch comprises a shell cover and comprises at least two cover shells, wherein one of the cover shells forms a roll cover shell and another of the cover shells forms a pitch cover shell. The roll cover shell is designed and/or arranged to compensate for the roll movement of the roll-pitch assembly, ensuring that the roll-pitch assembly is covered in all roll settings.

Similarly, the pitch cover shell is designed and/or arranged to compensate for the pitch movement of the roll-pitch assembly, ensuring that the roll-pitch assembly is covered in all pitch settings. The shell cover serves multiple purposes, including concealing the roll-pitch assembly and providing pinch protection for hands. This is crucial for ensuring the safety of both patients and medical staff, preventing accidental injuries during adjustments.

The cover shells are typically made from durable, lightweight materials, for example such as high-density polyethylene (HDPE) or medical-grade plastics. These materials are chosen for their strength, ease of cleaning, and resistance to wear and tear. Additionally, they must be resistant to ionizing radiation to maintain their integrity and functionality in a radiotherapy environment.

The cover shells are preferably configured as hard cover cases. Favorably, the cover shells are resistant to ionizing radiation and/or invisible in X-ray images. In particular, the cover shells are based on and/or made of carbon fiber composites, polycarbonates, Boron Nitride Nanotubes (BNNTs), Polyetherimid (PEI), High-Density Polyethylene (HDPE), Polyetheretherketon (PEEK), Polyimid (PI), Polyamid (PA), Polysulfon (PSU) and/or graphites. These materials ensure that the hard cover cases are both durable and suitable for use in environments with ionizing radiation, while also being compatible with X-ray imaging requirements. It's also possible to use other materials, such as polystyrene produced through the low-pressure process or polyamide with fiber-reinforced inserts produced through the high-pressure process.

In particular, the roll cover shell and pitch cover shell are designed to move in coordination with the roll-pitch assembly. This ensures that the assembly remains covered regardless of its position. Optionally, the roll cover shell compensates for the roll movement by rotating along with the roll axis, while the pitch cover shell compensates for the pitch movement by tilting along with the pitch axis. The cover shells can be fixed to the roll-pitch assembly with flexible joints that allow for smooth movement, ensuring that the shells move seamlessly with the assembly, providing continuous coverage. A prefered configuration involves telescopic shells that can extend and retract as needed, allowing the shells to adjust their length to cover the assembly fully, regardless of its position. The cover shells can also be segmented, with each segment capable of independent movement, allowing for more precise adjustments and ensuring that the assembly is always covered, even during complex movements.

The roll cover is designed to adjust dynamically during a roll and/or pitch movement of the roll-pitch assembly. For a roll movement it can involve a mechanism where the roll cover shortens on one side or partially slides into another cover element and/or shell, while on the opposite side, it extends or deploys to maintain continuous coverage of the roll-pitch assembly.

Especially, during a roll movement, the roll cover shell on the side that is moving downward will retract. This retraction can be achieved through a telescopic design, where the cover segments and/or shells slide into each other, or through a flexible joint mechanism that allows the cover to fold or collapse. On the opposite side, the roll cover will extend to compensate for the upward movement. This extension can be facilitated by a spring-loaded mechanism, a motorized actuator that pushes the cover outward or by connecting the cover parts to the moving frames, ensuring that the roll-pitch assembly remains fully covered. The pitch movement can involve an analogous and/or similar mechanism.

The shell cover is designed to meet stringent safety and hygiene standards. It provides a barrier that prevents accidental contact with the moving parts of the roll-pitch assembly, reducing the risk of injury. The materials used are easy to clean and disinfect, ensuring that the patient couch remains hygienic and suitable for medical use. Overall, the shell cover with its roll and pitch cover shells is a critical component of the patient couch, providing safety, functionality, and durability in a radiotherapy setting.

Preferably, the cover shells are movable relative to each other in the vertical direction. This feature allows the cover shells to adjust their position to accommodate the movements of the roll-pitch assembly, ensuring continuous coverage and protection. For example, the cover unit and/or cover shells can be designed to telescope into each other. In this configuration, the cover unit consist of multiple cover shells as segments that can slide into one another, similar to the sections of a telescope.

While the cover shells are movable relative to each other in the vertical direction, they are in particular fixed relative to each other in the horizontal plane. This means that the cover shells maintain their alignment and position horizontally, ensuring stability and preventing any lateral movement that could compromise the coverage or protection of the roll-pitch assembly.

The cover shells are preferably designed to be slidable into and out of each other to compensate for a pitch and/or roll movement. This feature allows the cover shells to dynamically adjust their position, ensuring continuous coverage of the roll-pitch assembly during its movements. For instance, in the case of a pitch movement, the cover shells can slide vertically relative to each other. When the roll-pitch assembly tilts forward or backward, the cover shells on one side can retract by sliding into the adjacent cover shell, while the cover shells on the opposite side extend by sliding out. This telescopic mechanism ensures that the roll-pitch assembly remains covered at all times, regardless of its pitch angle. Similarly, for a roll movement, the cover shells can slide horizontally relative to each other. As the roll-pitch assembly tilts to the left or right, the cover shells on the descending side can retract by sliding into the adjacent cover shell, while the cover shells on the ascending side extend by sliding out. This ensures that the roll-pitch assembly is continuously covered during the roll movement.

Preferably, the cover shells are designed to be receivable into each other to compensate for a pitch and/or roll movement. This means that the cover shells can nest within each other, providing a compact and efficient way to adjust their length. For example, during a pitch movement, the cover shells on one side can nest into the adjacent cover shell, reducing their overall length, while the cover shells on the opposite side extend by sliding out. This nesting mechanism ensures that the cover shells can adjust their length dynamically, providing continuous coverage of the roll-pitch assembly. Furthermore the covers can be designed in such a way that liquids run off from the top downwards and do not enter the interior.

Optionally, at least one of the cover shells has a projection, and a cover shell that is received by or receives the cover shell has a receiving section corresponding to the projection. The projection and the receiving section form a guidance system and/or interlock in such a way that the vertical movement is limited. The projection on one cover shell can be designed to fit precisely into the receiving section of the adjacent cover shell. This mechanism ensures that the cover shells move together in a controlled manner, providing stability and preventing unintended vertical displacement. The guidance system formed by the projection and the receiving section allows for smooth and precise adjustments, ensuring that the cover shells maintain continuous coverage of the roll-pitch assembly during its movements.

The projection can be a ridge or a rail that extends along the edge of the cover shell, while the receiving section can be a corresponding groove or channel on the adjacent cover shell. When the cover shells are assembled, the projection slides into the receiving section, creating a secure interlock that guides the vertical movement. This design ensures that the cover shells move in unison, maintaining their alignment and preventing any gaps or misalignment that could compromise the coverage of the roll-pitch assembly. The materials used for the projection and receiving section are typically the same as those used for the cover shells, such as high-density polyethylene (HDPE) or medical-grade plastics. These materials provide the necessary strength and durability for the interlocking mechanism, ensuring long-term reliability and functionality.

In a preffered embodiment, one of the cover shells forms an intermediate cover shell, wherein the intermediate cover shell is arranged between the roll cover shell and the pitch cover shell. The intermediate cover shell is designed and/or arranged to compensate for a roll and a pitch movement at least partially. The intermediate cover shell serves as a transitional element that bridges the roll cover shell and the pitch cover shell. This intermediate cover shell is for ensuring continuous coverage and smooth movement of the cover system during both roll and pitch adjustments. By being positioned between the roll cover shell and the pitch cover shell, the intermediate cover shell can adapt to the combined movements of the roll-pitch assembly, providing flexibility and stability. The intermediate cover shell is preferably designed to move in coordination with both the roll and pitch cover shells. It can slide or telescope into and out of the adjacent cover shells, allowing it to extend or retract as needed to accommodate the movements of the roll-pitch assembly.

Particularly, the roll-pitch assembly comprises a base frame and a pitch frame, wherein the base frame is fixedly connected to the base section and/or rests on the base section. The pitch frame is pivotable about the pitch axis, wherein the pitch axis rests on the base frame and/or the pitch axis is guided through an opening of the base frame. The base frame and pitch frame form a structural framework, which can be configured as a frame, box, or chassis. These frames are designed to be rigid and stable, providing a solid foundation for the roll-pitch assembly. They can be constructed from materials such as high-strength plastics or metals, including aluminum or steel, to ensure durability and resistance to mechanical stress. The axes, particularly the pitch axis, are designed as rotational axes. These axes are mechanical components that pass through specified parts of the frames, such as openings or bores. The pitch axis, for example, may be guided through an opening in the base frame, allowing the pitch frame to pivot smoothly around this axis. The axes can be supported by one or more bearings, which facilitate smooth and controlled rotational movement. These bearings can be ball bearings, roller bearings, or other types suitable for the specific mechanical requirements of the roll-pitch assembly.

The base frame is preferably securely attached to the base section of the patient couch, either through fixed connections such as bolts or welding, or by resting on the base section with a stable interface. This ensures that the base frame remains stationary and provides a reliable support structure for the pitch frame. The pitch frame, pivotable about the pitch axis, allows for precise adjustments of the couch board section's angle. This pivoting mechanism is essential for achieving the desired patient positioning during radiotherapy. The pitch axis, guided through the base frame, ensures that the pitch frame can move freely and accurately, maintaining the stability and alignment of the entire assembly.

Preferably, the roll-pitch assembly comprises a roll frame, wherein the roll frame and the roll axis are pivotable. The roll axis rests on the pitch frame and/or is guided through an opening in the pitch frame so that the roll axis follows a pivoting of the pitch frame about the pitch axis. The roll frame forms a structural component that supports the roll axis and allows for its pivoting movement. This frame can be constructed from rigid materials such as high-strength plastics or metals, including aluminum or steel, to ensure durability and stability. The roll frame is designed to pivot around the roll axis, enabling the couch board section to tilt laterally. The roll axis, which is a rotational axis, is supported by the pitch frame. This means that the roll axis is either directly mounted on the pitch frame or passes through an opening in the pitch frame. The opening can be a precisely machined bore or slot that allows the roll axis to rotate smoothly. Bearings may be used to support the roll axis, providing low-friction movement and ensuring precise control over the pivoting action. These bearings can be ball bearings, roller bearings, or other suitable types, depending on the mechanical requirements.

Optionally, a mechanical connection of the cover unit is made via the top and bottom cover shell in the vertical direction, wherein the top cover shell is mechanically connected to the couch board section and the bottom cover shell is mechanically connected to the base section. The top cover shell is designed to move in unison with the couch board section. This mechanical connection ensures that any vertical adjustments of the couch board section are mirrored by the top cover shell, maintaining continuous coverage and protection of the roll-pitch assembly. The connection can be achieved through various means, such as brackets, hinges, or sliding mechanisms, which securely attach the top cover shell to the couch board section. These connections are designed to be robust and durable, capable of withstanding repeated movements without compromising the integrity of the cover unit. Similarly, the bottom cover shell is mechanically connected to the base section. This connection ensures that the bottom cover shell remains stationary relative to the base section, providing a stable foundation for the cover unit.

Especially, the cover shells are designed to be form-stable, providing a rigid and durable hard cover for the roll-pitch assembly. Mechanically, the cover shells are for example constructed from high-strength materials such as high-density polyethylene (HDPE) or medical-grade plastics, which ensure their structural integrity and resistance to deformation under stress.

The invention is also related to a radiotherapy device comprises the patient, a control unit for controlling and/or adjusting the roll-pitch assembly, and a linear accelerator (LINAC) or a radiation source for generating therapeutic radiation. The patient couch is arranged and/or designed so that a patient can be positioned at a desired irradiation angle.

The LINAC is a component of the radiotherapy device, responsible for generating high-energy X-rays or electron beams used to treat cancerous tissues. The LINAC connected with a gantry, which is a rotating structure that allows the radiation source to move around the patient. This movement enables the delivery of radiation from multiple angles, ensuring precise targeting of the tumor while minimizing exposure to surrounding healthy tissues.

The patient couch, in combination with the LINAC, plays a vital role in patient positioning and treatment accuracy. The couch is designed to be highly adjustable, allowing for precise control of the patient's position. The roll-pitch assembly, controlled by the control unit, enables the couch to pivot around the roll and pitch axes, ensuring that the patient can be positioned at the exact angle required for effective irradiation. The control unit is responsible for managing the movements of the roll-pitch assembly, allowing for fine adjustments to the patient's position. This ensures that the patient remains in the correct position throughout the treatment, even if minor adjustments are needed during the session. The control unit can be integrated with the LINAC's control system, providing a seamless interface for the radiotherapy technician to manage both the radiation delivery and patient positioning.

A further objective of the invention relates to the use of the patient couch for positioning a patient at an adjustable irradiation angle in a radiotherapy device. The patient couch, equipped with a roll-pitch assembly and optinally controlled by a dedicated control unit, allows for precise adjustments of the patient's position. This ensures optimal alignment with the therapeutic radiation beam generated by a linear accelerator (LINAC) or other radiation sources. The patient couch's ability to pivot around roll and pitch axes, combined with its vertical and horizontal adjustability, enables accurate positioning at various irradiation angles. This enhances the effectiveness of radiotherapy treatments by allowing for targeted delivery of radiation to the tumor while minimizing exposure to surrounding healthy tissues. The integration of the patient couch with the radiotherapy device ensures seamless operation and improved patient outcomes.

Features disclosed in connection with particular embodiments of the invention may be combined with features of other embodiments, even if such combinations are not explicitly stated. In particular, features pertaining to different claim categories, such as methods, devices, and uses, are considered mutually transferable, provided that such a combination is technically feasible and does not deviate from the core inventive concept. These combinations are within the scope of the invention as disclosed herein.

The invention is not limited to the specific embodiments described herein. Variations, modifications, and alternative configurations that fall within the scope of the appended claims and are equivalent to the disclosed embodiments are also encompassed by the invention. Unless otherwise stated, terms used in the claims and description should not be interpreted in a restrictive manner, but rather in accordance with their broadest technically feasible meaning, as understood by a person skilled in the art.

The invention aims to address one or more technical problems as identified herein or as may become apparent to a person skilled in the art. However, the invention is not limited to solving the explicitly stated problems but also encompasses any other advantages or improvements achieved through its implementation.

Further embodiments, functionalities, and effects of the invention are apparent from the accompanying figures and their descriptions. The figures illustrate:
- Figure 1: a radiotherapy device with a patient couch;
- Figure 2: a section of the patient couch, comprising a roll-pitch-assembly;
- Figure 3a, b, c, d: the roll-pitch assembly after different roll- and/or pitch movements;
- Figure 4: an embodiment of the cover unit;
- Figure 5 a-i: section of the patient couch for various roll and/or pitch movements.

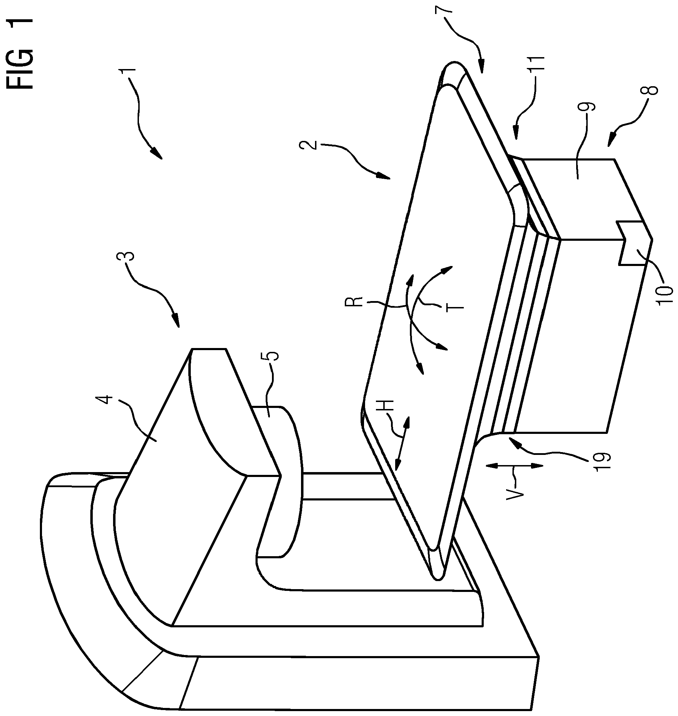

Figure 1 shows a radiotherapy device 1. The radiotherapy device 1 comprises a patient couch 2 and a linear accelerator 3. The linear accelerator 3 includes a gantry 4 and a beam outlet 5. The linear accelerator 3 and the patient couch 2 are arranged such that a patient positioned on the patient couch 2 can be treated with radiation from the linear accelerator 3 during radiotherapy treatment.

The patient couch 2 comprises a couch board section 7 and a base section 8. The couch patient section 7, especially the upper part of the patient couch 2, provides accommodation and fixation for the patient. The couch board section 7 preferably includes a flat or curved surface and defines a horizontal plane. The surface of the couch board section 7 is preferably transparent to x-ray and high-energy photon beams.

The base section 8, particularly the lower part of the patient couch 2, connects the patient couch 2 to the ground and may include mechanisms for vertical V and horizontal H movement of the patient. The couch board section 8 include mechanisms for rotational R and tilting T movements, allowing optimal positioning of the patient for treatment. The base section 8 is preferably covered and/or shielded by a cover 9.

The radiotherapy device 1 and patient couch 2 include a control unit 10, configured to control and/or steer the rotational and tilting movements, as well as the horizontal and vertical movements of the patient couch 2. The control unit 10 may also be integrated with the control unit of the linear accelerator 3, ensuring coordinated positioning and treatment of the patient with radiation.

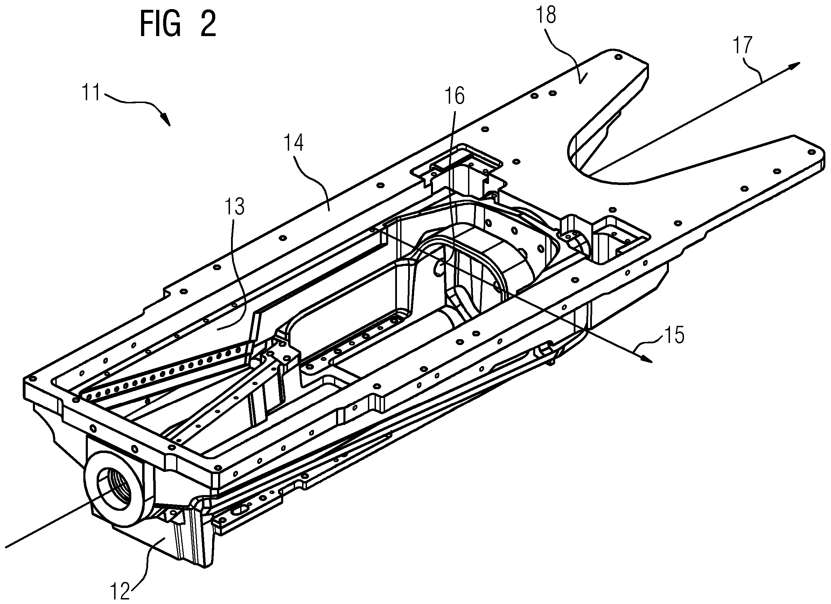

Figure 2 shows a section of the roll-pitch assembly 11. The roll-pitch assembly 11 is positioned between the couch board section 7 and the base section 8. The depicted assembly is modular and includes a base frame 12, a pitch frame 13, and a roll frame 14. The frames 12 to 14 form rigid structures, typically made of metallic materials such as steel or made of plastics or graphite. The frames are partially nested within one another.

The base frame 12 forms the lowest section in the vertical direction V and is fixed and/or firmly mounted. The base frame 12 sits on the base section 8. In other words, the base frame 12 is connected to the floor through the base section 8. As part of a height adjustment mechanism, particularly through a mechanism in the base section 8, the base frame 12 can also be adjusted in height and/or longitutinal direction. The base frame 12, for example, forms a trapezoidal frame in top view. To minimize weight and improve radiation transparency, the edges of the frame are essentially solid, so that in top view, the interior of the frame is only reinforced by individual struts.

The pitch frame 13 at least partially surrounds the base frame 12. The pitch frame 13 is designed in the shape of a frame and is essentially rectangular in a top view. The pitch frame 13 is pivotable and/or rotatable about a pitch axis 15. The pitch axis 15 forms in particular a mechanical axis, for example a rod-shaped or cylindrical metal axis. The pitch frame 13 is firmly connected to the pitch axis 15, in particular in a material-locking manner, for example welded, form-locking, or force-locking.

The pitch axis 15 extends through the base frame 12 and/or is rotatably received or mounted on the base frame. For this purpose, the base frame 12 comprises two opposite base frame openings 16, through which the pitch axis 15 extends. Specifically, the base frame openings 16 may include a bearing, in particular a rolling bearing, for mounting the pitch axis 15. The pitch axis thus rests on the base frame 12. In other words, by connecting the base frame and the pitch frame via the pitch axis, a relative tilting of the base frame 12 and the pitch frame 13 with respect to each other is possible. This relative movement enables the pitch movement of the patient couch 2.

The roll-pitch assembly 11 comprises a roll frame 14, which is essentially shaped like a frame and is essentially trapezoidal or rectangular in a top view. The roll frame 14 has a support section 18 on its upper side for the couch board section 7. The roll frame 14 at least partially surrounds the pitch frame 13. The roll frame 14 is pivotable and/or rotatable about a roll axis 17.

The roll axis 17 is essentially a mechanical rotation axis and is oriented in the longitudinal direction of the patient couch. The roll axis 17 rests on the pitch frame 13. In particular, the roll axis 17 extends through an opening 18 in the pitch frame. Here, the axis may be mounted by means of a bearing. Through this arrangement, the roll axis is also tilted during a pitch movement of the pitch frame 13, i.e., the roll axis 17 follows the pitch movement. To prevent the ingress of dirt and liquids, as well as to prevent hands or objects from reaching in and getting pinched, the roll-pitch assembly 11 is surrounded by a cover unit 19.

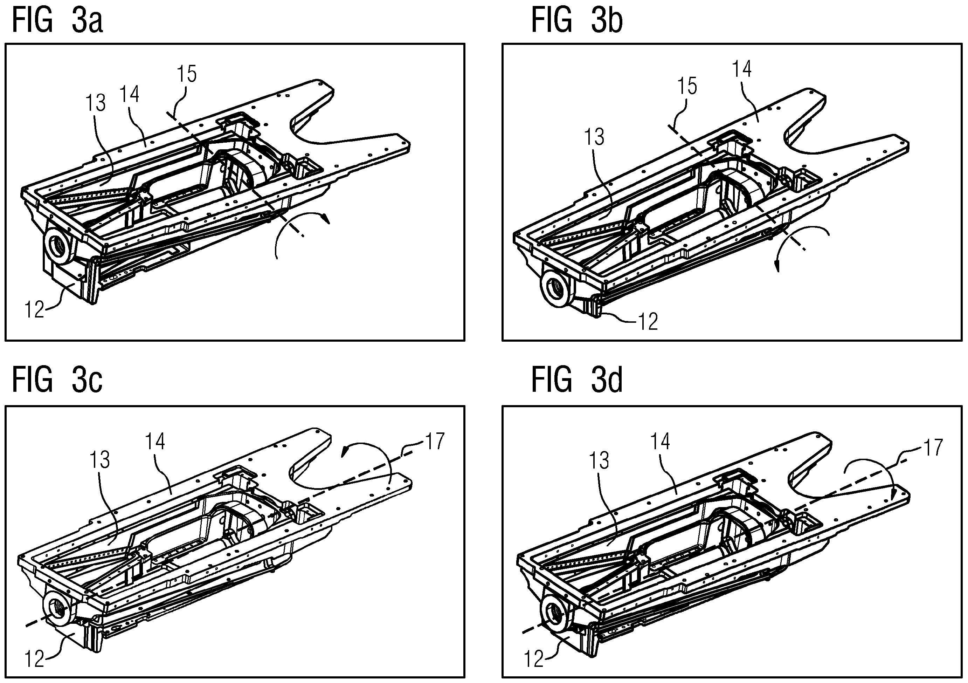

Figure 3a depicts the pitch frame 13 and roll frame 14 in a pitched backward position, also called head-down position, showing the rear segment inclined downward and the front segment tilted upward. Consequently, the roll axis 17 tilts from the front-top to the rear-bottom.

Figure 3b demonstrates the same frames 12-14 pitched forward, also calles head-up position, with the front parts tilted downwards and the rear sections upwards. The roll axis 17 inclines from the front-bottom to the rear-top.

Figure 3c shows a leftward roll movement, with the left side of the roll frame 14 tilting downwards while the right side tilts upwards.

Figure 3d depicts a rightward roll movement, showing the right side of the roll frame 14 tilting downwards and the left side upwards. The roll-pitch assembly 11, covered by the cover unit 19, is shown adapting to these movements.

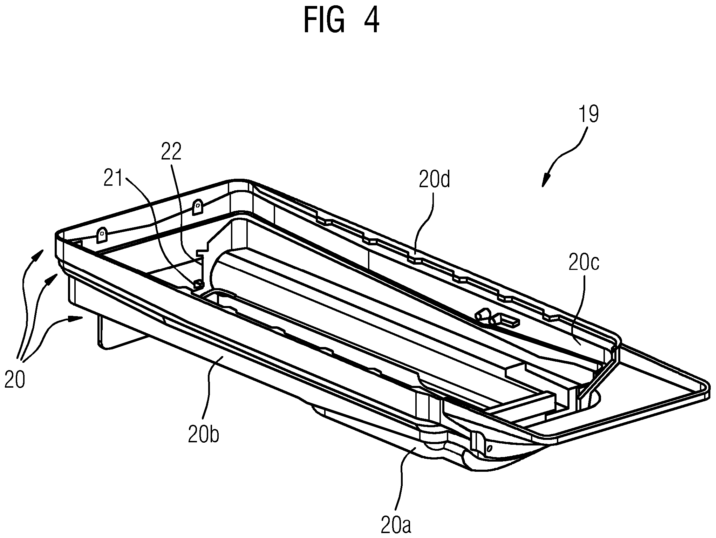

Figure 4 illustrates an exemplary embodiment of the cover unit 19, which surrounds the roll-pitch assembly 11, particularly from the sides. The cover unit 19 connects to the base section 8 and the couch board section 7, forming a protective housing made up of multiple cover shells 20. These cover shells 20, made from rigid plastic, are telescopically connected, allowing for relative vertical movement.

The base cover shell 20a is mechanically attached to the base frame 12, while the pitch cover shell 20b, mechanically connected to the base cover shell 20a, features a projection 21 fitting into a receiving section 22 of the base cover shell 20a, allowing limited movement while ensuring a secure fit.

An intermediate cover shell 20c, telescopically connected to and/or interacting with both the pitch cover shell 20b and the roll cover shell 20d, enables seamless movement. The intermediate cover schell 20c is mechanically attached to the pitch frame 13.

The roll cover shell 20d, telescopically connected to the intermediate cover shell 20c, ensuring secure movement.

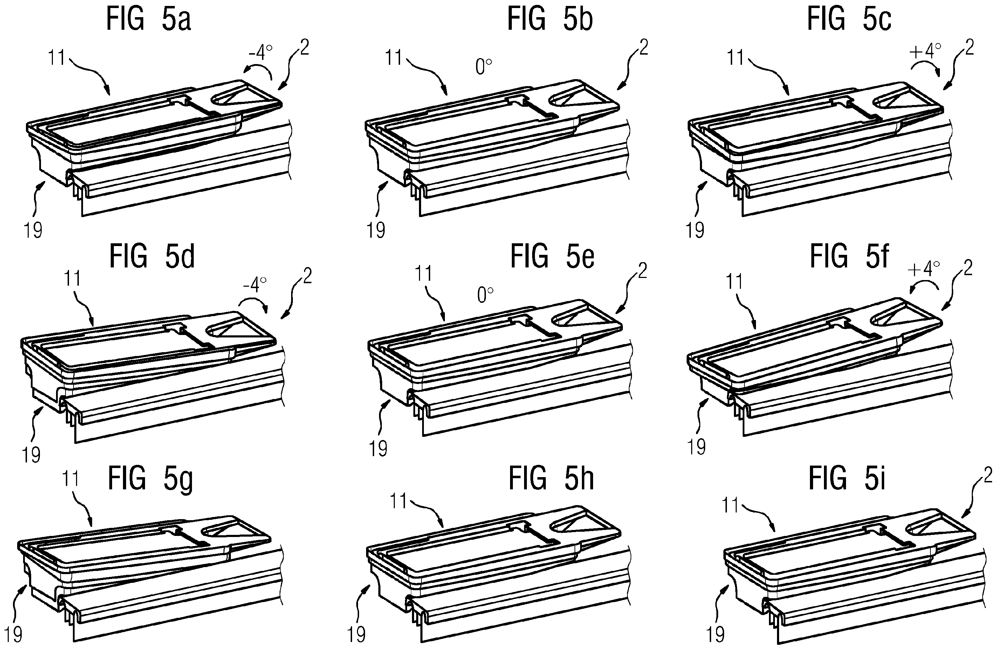

Figures 5a-c show the section for various roll movements. 5b illustrates the neutral position at 0° roll, 5a depicts a 4° backward roll, and 5c shows a 4° forward roll. During these movements, the roll cover shell 20d adapts by receiving or extending the intermediate cover shell 20c.

Figures 5d-f display the section for various pitch movements. 5e shows the neutral position at 0° pitch, 5d depicts a 4° rightward pitch also called head-down, and 5f shows a 4° leftward pitch also called head-up. The pitch cover shell 20b adapts similarly to the base cover shell 20a.

Figures 5g-i depict combinations of roll and pitch movements. 5h shows the neutral position at 0° roll and pitch, 5g illustrates a 4° rightward pitch also called head-down and 4° backward roll also called head-up, and 5i shows a 4° leftward pitch and 4° forward roll. The telescopically nested shells of the cover unit 19 effectively adapt to these combined movements, ensuring the mechanical parts remain covered and protected.

## Claims

1. Patient couch (2) for positioning and/or supporting a patient for radiotherapy, comprising:
- a couch board section (7) for accommodating the patient,
- a base section (8) for attachment to the floor,
- a roll-pitch assembly (11) arranged between the base section (8) and the couch board section (7), designed to pivot the couch board section (7) about a roll axis (17) and/or a pitch axis (15),
**characterized by** a cover unit (19) for concealing the roll-pitch assembly (11) and/or as a pinch protection for hands, wherein the cover unit (19) forms a shell cover and comprises at least two cover shells (20), wherein one of the cover shells (20) forms a roll cover shell (d) and another of the cover shells (20) forms a pitch cover shell (20b), wherein the roll cover shell (20b) is designed and/or arranged to compensate a roll movement of the roll-pitch assembly (11), wherein the pitch cover shell (20b) is designed and/or arranged to compensate a pitch movement of the roll-pitch assembly (11).

2. Patient couch (2) according to claim 1, **characterized in that** the cover shells (20) are movable relative to each other in the vertical direction (V).

3. Patient couch (2) according to claim 1 or 2, **characterized in that** the cover shells (20) are designed to be slidable into and out of each other to compensate for a pitch and/or roll movement and/or that the cover shells (20) are designed to be receivable into each other to compensate for a pitch and/or roll movement.

4. Patient couch (2) according to claim 2 or 3, **characterized in that** one of the cover shells (20) has a projection (21) and a cover shell (20) that is received by or receives the cover shell has a receiving section (22) corresponding to the projection (21), wherein the projection (21) and the receiving section (22) form a guidance system and/or interlock in such a way that the vertical movement is limited.

5. Patient couch (2) according to one of the preceding claims, **characterized in that** one of the cover shells (20) forms an intermediate cover shell (20c), wherein the intermediate cover shell (20c) is arranged between the roll cover shell (20d) and the pitch cover shell (20), wherein the intermediate cover shell (20c) is designed and/or arranged to compensate a roll and a pitch movement at least partially.

6. Patient couch (2) according to one of the preceding claims, **characterized in that** the roll-pitch assembly (11) comprises a base frame (12) and a pitch frame (13), wherein the base frame (12) is fixedly connected to the base section (8) and/or rests on the base section (8), wherein the pitch frame is pivotable about the pitch axis (15), wherein the pitch axis (15) rests on the base frame (12) and/or the pitch axis (15) is guided through an opening of the base frame.

7. Patient couch (2) according to claim 6, **characterized in that** the roll-pitch assembly (11) comprises a roll frame (14), wherein the roll frame and the roll axis are pivotable, wherein the roll axis rests on the pitch frame (13) and/or the roll axis (17) is guided through an opening in the pitch frame (13) so that the roll axis (17) follows a pivoting of the pitch frame (13) about the pitch axis (15).

8. Patient couch (2) according to one of the preceding claims, **characterized in that** a mechanical connection of the cover unit (19) is made via the top and bottom cover shell (20) in the vertical direction (V), wherein the top cover shell (20) is mechanically connected to the couch board section (7) and the bottom cover shell (20) is mechanically connected to the base section (8).

9. Patient couch (2) according to one of the preceding claims, **characterized in that** the cover shells (20) are designed to be rigid and/or form a hard shell.

10. Radiotherapy device (1) comprising a patient couch (2) according to one of the preceding claims, a control unit (10) for controlling and/or adjusting the roll-pitch assembly (11), and a linear accelerator (3) or a radiation source for generating therapeutic radiation, wherein the patient couch (2) is arranged and/or designed so that a patient can be positioned in a desired irradiation angle.

11. Use of a patient couch (2) according to one of claims 1 to 9 for positioning a patient at an adjustable irradiation angle at a linear accelerator (2) and/or in a radiotherapy device (1).
